# FASCICULE DE BREVET EUROPEEN

(11) **EP 1 617 836 B1**
(45) Date de publication et mention de la délivrance du brevet: **30.11.2011**
(21) Numéro de dépôt: 04742843.8
(22) Date de dépôt: 09.04.2004
(51) Int. Cl.: A61K 31/351, A61K 31/70, A61K 36/63, A23L 1/29, A61P 19/08

(54) **COMPOSITION NUTRITIONELLE OU THERAPEUTIQUE CONTENANT LE COMPOSE OLEUROPEINE OU L'UN DE SES DERIVES**
NAHRUNGS- ODER THERAPEUTISCHE ZUSAMMENSETZUNG, ENTHALTEND OLEUROPEIN ODER SEINE DERIVATE
NUTRITIONAL OR THERAPEUTIC COMPOSITION CONTAINING THE COMPOUND OLEUROPEINE OR ONE OF THE DERIVATIVES THEREOF

(30) Priorité: 11.04.2003 FR 0304584
(43) Date de publication de la demande: 25.01.2006
(73) Titulaire: INSTITUT NATIONAL DE LA RECHERCHE AGRONOMIQUE (INRA), 75338 Paris Cédex 07 (FR)
(72) Inventeur: COXAM, Véronique, F-63122 CEYRAT (FR); SKALTSOUNIS, Leandros, GR-15127 MELISSIA (GR); PUEL, Caroline, 63540 Romagnat (FR); MAZUR, André, F-63800 COURNON D'AUVERGNE (FR)
(74) Mandataire: Catherine, Alain
(86) Numéro de dépôt international: PCT/FR2004/050156
(87) Numéro de publication internationale: WO 2004/091591

(56) Documents cités:
- WO-A-01/76579
- WO-A-96/14064
- WO-A-03/068171
- OWEN R W ET AL: "THE ANTIOXIDANT/ANTICANCER POTENTIAL OF PHENOLIC COMPOUNDS ISOLATED FROM OLIVE OIL" EUROPEAN JOURNAL OF CANCER, PERGAMON PRESS, OXFORD, GB, vol. 36, no. 10, 2000, pages 1235-1247, XP001093631 ISSN: 0959-8049
- DATABASE WPI Section Ch, Week 200268 Derwent Publications Ltd., London, GB; Class B04, AN 2002-630239 XP002266350 & JP 2002 128678 A (TAMA SEIKAGAKU KK) 9 mai 2002 (2002-05-09)

## Description

### DOMAINE DE L'INVENTION

L'invention se rapporte au domaine du maintien ou du rétablissement de l'équilibre du métabolisme osseux chez l'homme ou l'animal, notamment pour la prévention ou le traitement de troubles liés à un déséquilibre du métabolisme osseux grâce à l'apport nutritionnel ou à l'administration thérapeutique d'une composition stimulant la formation de l'os et/ou inhibant la résorption osseuse.

### ETAT DE LA TECHNIQUE.

L'os n'est pas un tissu statique. L'os subit un remodelage constant par destruction et synthèse *de novo* du tissu osseux dans un processus complexe impliquant deux types principaux de cellules, respectivement les ostéoblastes qui produisent du tissu osseux nouveau et les ostéoclastes qui détruisent l'os.

Les activités de ces cellules sont régulées par un grand nombre de cytokines et de facteurs de croissance, dont la plupart ont été identifiés et clonés, comme cela est décrit dans la revue générale de Mundy (Mundy, G.R., 1996, Clin. Orthop., vol.324 :24-28 ; Mundy, G.R., 1993, J.Bone Miner Res, vol.8 : S 505 - S 510).

Les ostéoblastes, cellules responsables de la formation de l'os, se différencient à partir de cellules précurseurs et expriment et secrètent des protéines structurales telles que le collagène de type I, ainsi que des enzymes (phosphatase alcaline) et de nombreux peptides régulateurs et les BMP (Bone Morphogenetic proteins) (Stein G. et al., 1990, Curr. Opin Cell Biol. vol.2: 1018-1027; Harris S et al. ,1994, J. Bone Miner Res Vol.9:855-863).

Les ostéoclastes sont des cellules multinucléées qui sont responsables de la perte osseuse, dans un processus communément désigné la résorption osseuse.

En période de croissance de l'homme ou l'animal, l'activité de formation de l'os prédomine : c'est l'acquisition du capital osseux.

A l'âge adulte, chez l'homme ou l'animal, l'action équilibrée des ostéoblastes et des ostéoclastes permet un maintien dans le temps de la masse osseuse et assure simultanément un remodelage du tissu osseux par résorption et synthèse *de novo* de l'os.

Cependant, avec l'âge, il se produit un déséquilibre dans le processus de remodelage osseux, qui aboutit à une perte d'os, qui est désignée ostéopénie.

L'ostéopénie liée à l'âge est un phénomène universel, non pathologique en soi, mais qui constitue le terrain de l'ostéoporose puisque la réduction de la masse osseuse est le facteur étiologique essentiel dans la genèse de cette affection, ce qui n'exclut cependant pas l'influence d'autres paramètres tels que, par exemple, l'architecture du squelette ou la propension à chuter.

En effet, exacerbée, cette ostéopénie aboutit au risque fracturaire (densité minérale au-dessous de laquelle le moindre choc est susceptible de provoquer une fracture) qui détermine l'apparition de l'ostéoporose. On peut donc concevoir l'ostéoporose (post-ménopausique ou sénile) comme une pathologie infantile dont la prophylaxie pourrait être basée aussi bien (i) sur une optimisation du capital osseux (acquis au cours de la croissance de l'individu), que (ii) sur un ralentissement de la perte osseuse liée au vieillissement.

On observe une grande hétérogénéité dans la valeur du pic de masse osseuse selon les individus, par suite de variations dans le processus de croissance osseuse dès le plus jeune âge. Ainsi, la masse osseuse maximale atteinte avant 30 ans, aussi désignée pic de masse osseuse, est-elle très variable d'un individu à l'autre. Avec l'âge, les individus possédant une valeur faible de pic de masse osseuse sont désavantagés.

Par conséquent une prise en charge précoce de l'individu doit être privilégiée, puisque les deux phases critiques pour le capital osseux sont :
- la période de croissance, permettant l'acquisition de la masse osseuse maximale (pic de masse osseuse) ;
- le vieillissement, conditionnant la vitesse de perte de la masse osseuse.

La prévention de l'ostéoporose ne doit donc plus être restreinte à l'individu âgé.

Parmi les désordres pathologiques liés à un déséquilibre du métabolisme osseux, on peut citer notamment l'ostéoporose, la maladie de Paget, la perte osseuse ou l'ostéolyse observée à proximité d'une prothèse, les maladies de l'os métastatique, l'hypercalcémie due à un cancer, les myélomes multiples, et les maladies périodontales.

Certains des troubles ou pathologies du métabolisme osseux peuvent être provoqués après une immobilisation de longue durée, par exemple une hospitalisation , ou encore après un séjour en apesanteur.

Il existe d'autres facteurs susceptibles d'accroître la perte osseuse conduisant à l'ostéoporose, telle que la consommation de cigarettes, l'abus d'alcool, un mode de vie sédentaire, un faible apport en calcium, une alimentation déséquilibrée ou encore une carence en vitamine D.

Parmi les troubles liés à une résorption osseuse anormale , le plus commun est l'ostéoporose, dont la manifestation la plus fréquence est observée chez les femmes, après le début de la ménopause. L'ostéoporose est une maladie squelettique systémique caractérisée par une réduction de la masse osseuse et une détérioration de la microarchitecture du tissu osseux, associées à une augmentation de la fragilité de l'os et de sa susceptiblité à la fracture.

Selon la classification clinique retenue par l'Organisation Mondiale de la Santé (OMS), l'état de santé de l'os d'un individu est déterminé par la valeur de la densité minérale osseuse (DMO), telle que mesurée par ostéodensitométrie, comparée à une valeur normale prédéterminée.

Un individu possédant une valeur de DMO inférieure de moins de 1 DS (DS = déviation standard) par rapport à la valeur de DMO normale prédéterminée pour l'adulte jeune est considéré comme un individu « normal » (T-score > -1 DS, dans lequel « T-score » est la différence exprimée en nombre de déviations standards DS entre la DMO de l'individu et la valeur moyenne de la DMO d'une population adulte jeune de référence).

Un individu possédant une valeur de DMO comprise entre -1 et - 2,5 DS par rapport à celle de l'adulte jeune est classé comme un individu affecté d'une « ostéopénie » (-1 DS > T-score > -2,5 DS).

Un individu possédant une valeur de DMO inférieure à celle de l'adulte jeune de plus de 2,5 DS est classé comme individu affecté d'une ostéoporose (T-score < -2,5 DS).

Un individu possédant une valeur de DMO inférieure à celle de l'adulte jeune de plus de 2,5 DS et chez lequel la présence d'une ou plusieurs fractures a été décelée est classé comme un individu affecté d'une ostéoporose confirmée. (T-score < -2,5 DS + fractures).

On distingue deux types d'ostéoporose, respectivement de type I et de type II.

L'ostéoporose de type I est six fois plus fréquente chez la femme que chez l'homme. L'ostéoporose de type I survient surtout dans un sous-groupe de femmes ménopausées, âgées de 51 à 75 ans, et est caractérisée par une perte osseuse exagérée prédominant dans l'os trabéculaire. Les fractures des corps vertébraux et de l'extrémité inférieure de l'avant-bras en sont les complications habituelles. L'ostéoporose de type I est principalement liée à la carence hormonale oestrogénique de la ménopause, et dans une certaine mesure également de l'andropause.

L'ostéoporose de type II atteint une large population d'hommes et de femmes de plus de 70 ans et est associée à des fractures du col du fémur, de l'extrémité supérieure de l'humérus et du tibia, c'est à dire des sites osseux contenant à la fois de l'os cortical et de l'os trabéculaire. Les taux circulants de parathormone (PTH) sont souvent élevés. L'ostéoporose de type II est deux fois plus fréquente chez la femme.

Chez l'homme ou l'animal, il existe de nombreux états caractérisés par la nécessité d'accroître la formation osseuse. Par exemple, dans le cas de fractures osseuses, il est nécessaire de stimuler la croissance osseuse afin d'accélérer une réparation complète de l'os. Ce besoin est également présent dans les maladies périodontales, les maladies métastatiques de l'os, les maladies ostéolytiques et les états dans lesquels on recherche une réparation du tissu conjonctif, par exemple pour la cicatrisation ou la régénération des défauts ou des traumatismes du cartilage. On recherche également la stimulation d'une croissance osseuse dans le cas d'hyperparathyroïdisme primaire et secondaire, ainsi que dans l'ostéoporose associée au diabète et dans l'ostéoporose associée aux glucocorticoïdes, ainsi que dans l'ostéoporose androgénique chez l'homme.

Jusqu'à présent, les stratégies visant à rétablir une imprégnation oestrogénique (et androgénique chez l'homme) par hormonothérapie substitutive étaient fortement encouragées, notamment pour atténuer la cohorte des signes fonctionnels de la ménopause, et surtout réduire les risques vasculaires et osseux engendrés par la carence hormonale. Néanmoins, pour diverses raisons (contre-indications, réticence à prescrire des hormones, etc .), ce type de traitement prophylactique est appliqué à moins de 30% des femmes ménopausées. Cette abstention thérapeutique risque encore d'être aggravée par le climat de suspicion actuel autour du traitement hormonal, par suite de la publication récente de deux études américaines mettant en évidence un risque cardiovasculaire et cancéreux (HERS, 1998, 2002 ; WHI, 2002).
- Hulley S, Grady D, Bush T. Furberg C, Herrington D, Riggs B, Vittinghoff E, 1998 Randomized trial of estrogen plus progestin for secondary prévention of coronary heart disease in postmenopausal women. Heart and Estrogen/progestin Replacement Study (HERS) Research Group , JAMA 280(7) :605-13.
- Hulley S. Furberg C, Barrett-Connor E, Cauley J, Grady D, Haskell W. Knopp R, Lowery M, Satterfield S, Schrott H, Vittinghoff E, Hunninghake D, 2002 Non-cardiovascular disease outcomes during 6-8 years of hormone therapy: Heart and Estrogen/progestin Replacement Study follow-up (HERS II). Jama 288(1):58-66.
- Rossouw JE, Anderson GL, Prentice RL, LaCroix AZ, Kooperberg C, Stefanick ML, Jackson RD, Beresford SA, Howard BV, Johnson KC, Kotchen JM, Ockene J, 2002 Women's health initiative Risks and benefits of estrogen plus progestin in healthy post menopausal women. JAMA 288:321-333.

Les modulateurs sélectifs du récepteur oestrogénique (ou SERMs, pour « Selective Estrogenic Receptor Modulators »), tels que le raloxifène, sont également préconisés. Toutefois, ils restent inefficaces sur l'incidence des bouffées de chaleur.

On connaît également des stéroïdes synthétiques, comme la tibolone, qui possède une activité oestrogénique et progestative, avec une faible propriété androgénique, mais qui peut provoquer des leucorrhées, des vaginites, de la mastodynie et qui peut entraîner une prise de poids.

De fait, il existe aujourd'hui de nombreux composés actifs sur la stimulation de la formation de l'os ou l'inhibition de la résorption osseuse, parmi lesquels on peut citer la famille des polyphosphonates, comme l'etidronate ou l'alendronate (brevet européen n°EP 210 728, demande de brevet US publiée sous le n°2001/0046977), les oxazolidinones thioamide (demande de brevet US publiée sous le n°2002/0010341), ou encore les composés isoflavones (demande de brevet US publiée sous le n°2002/0035074). On a également proposé l'utilisation d'extrait d'une plante du genre *Lycopersicon* (demande de brevet US publiée sous le n°2002/0009510).

Bien qu'il existe aujourd'hui une grande diversité de composés actifs pour stimuler la formation osseuse et/ou inhiber la résorption osseuse, il existe un besoin constant pour de nouveaux composés actifs, notamment du fait du succès limité des traitements actuels.

De plus, compte-tenu du caractère chronique de certains états provoqués par un déséquilibre du métabolisme osseux, il existe un besoin pour de nouveaux composés actifs susceptibles de pouvoir être utilisés pendant une longue période du temps chez l'homme ou l'animal.

C'est pourquoi les professionnels de la santé, ainsi que les instances officielles de réglementation (rapport sur l'ostéoporose dans la Communauté Européenne, 1998), recommandent d'intégrer des thérapies validées complémentaires, voire alternatives. Une approche nutritionnelle (par exemple sous la forme d'une composition) répond tout à fait à ces critères.

### SOMMAIRE DE L'INVENTION.

De manière surprenante, le demandeur a montré que l'oleuropéine, qui est un composé contenu notamment dans les plantes de la famille des *oleaceae*, est capable d'agir sur le métabolisme osseux en freinant la déminéralisation de l'os.

Il est donc décrit une composition nutritionnelle et une composition pharmaceutique à usage humain ou vétérinaire comprenant, à titre de composé actif, l'oleuropéine ou l'un de ses dérivés.

Il est également décrit l'utilisation du composé oleuropéine ou de l'un de ses dérivés pour la fabrication d'une composition destinée à stimuler la minéralisation de l'os, en particulier la formation de l'os et/ou l'inhibition de la résorption osseuse chez l'homme ou l'animal. Selon un premier aspect, l'utilisation ci-dessus est caractérisée en ce que ladite composition est une composition nutritionnelle adaptée pour une administration orale.

Selon un second aspect, l'utilisation ci-dessus est caractérisée en ce que ladite composition est une composition pharmaceutique pour une administration orale, parentérale, intramusculaire ou intra-veineuse.

### DESCRIPTION DES FIGURES.

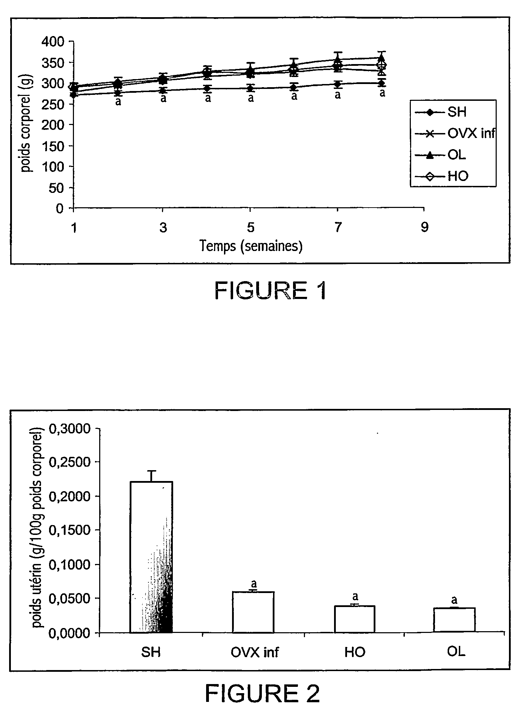

La **Figure 1** illustre l'évolution pondérale (i) des rates témoins (SH), (ii) des rates ovariectomisées et ayant subi une inflammation (OVX inf) ayant reçu un régime alimentaire témoin, (iii) des rates « OVX inf » ayant reçu un régime alimentaire contenant un extrait d'oleuropéine (OL) et (iv) des rates « OVX inf » ayant reçu un régime alimentaire contenant de l'huile d'olive (HO). En ordonnées : le poids corporel moyen des rates ; en abscisses : la durée du traitement suivant le moment de l'ovarlectomie.
La **Figure 2** illustre le poids utérin (i) des rates témoins (SH), (ii) des rates ovariectomisées et ayant subi une inflammation (OVX inf) ayant reçu un régime alimentaire témoin, (iii) des rates « OVX inf » ayant reçu un régime alimentaire contenant de l'huile d'olive (HO), et (iv) des rates « OVX inf » ayant reçu un régime alimentaire contenant un extrait d'oleuropéine (OL). En ordonnées : le poids utérin moyen des rates +/- l'erreur standard par rapport à la moyenne (SEM) ; en abscisses : chaque groupe de rates. Pour les lots OVX inf, HO et OL, *p<0,0001 par rapport au groupe témoin SH.

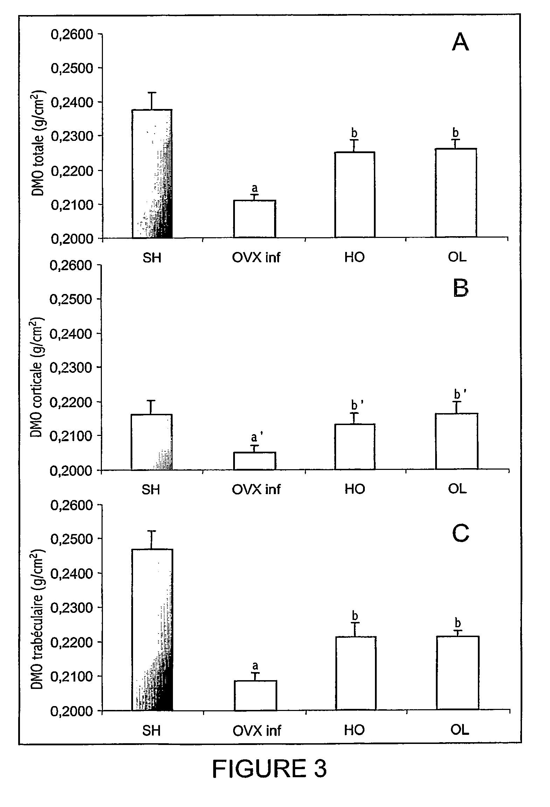

La **Figure 3** illustre la densité minérale osseuse totale (figure 3A), la densité minérale osseuse corticale (figure 3B) et la densité minérale osseuse trabéculaire (figure 3C), respectivement (i) des rates témoins (SH), (ii) des rates ovariectomisées et ayant subi une inflammation (OVX inf) ayant reçu un régime alimentaire témoin, (iii) des rates « OVX inf » ayant reçu un régime alimentaire contenant de l'huile d'olive (HO), et (iv) des rates « OVX inf » ayant reçu un régime alimentaire contenant un extrait d'oleuropéine (OL). En ordonnées : la densité minérale osseuse (DMO), exprimée en g/cm² +/- l'erreur standard par rapport à la moyenne (SEM) ; en abscisses : chaque groupe de rates. « a » : p<0,001 par rapport au groupe témoin SH. « a' » : p<0,01 par rapport au groupe témoin SH. « b » : p<0,005 par rapport au groupe OVX inf. « b' » : p<0,05 par rapport au groupe OVX inf.

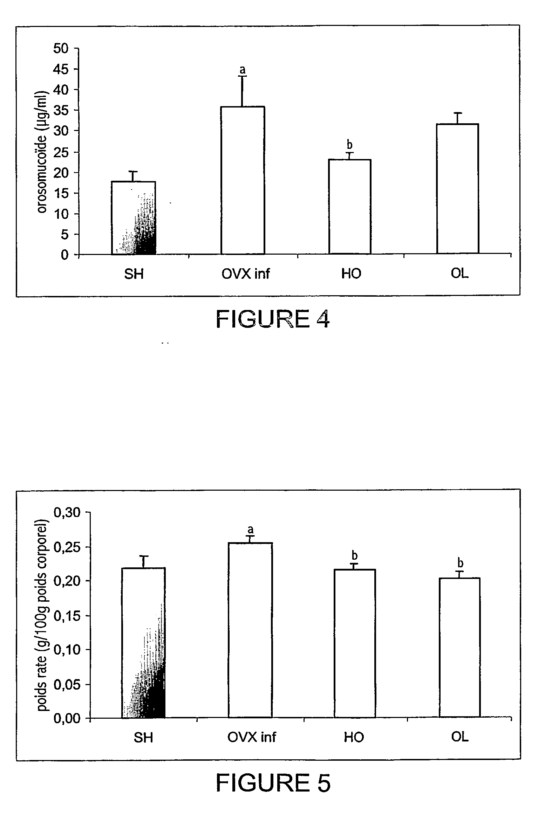

La **Figure 4** illustre la concentration plasmatique en orosomucoïde (i) des rates témoins (SH), (ii) des rates ovariectomisées et ayant subi une inflammation (OVX inf) ayant reçu un régime alimentaire témoin, (iii) des rates « OVX inf » ayant reçu un régime alimentaire contenant de l'huile d'olive (HO), et (iv) des rates « OVX inf » ayant reçu un régime alimentaire contenant un extrait d'oleuropéine (OL). En ordonnées : concentration d'orosomucoïde, exprimée en µg/ml, +/- l'erreur standard par rapport à la moyenne (SEM). En abscisses : en abscisses : chaque groupe de rates. « a » : p<0,05 par rapport au groupe témoin SH ; « b » : p<0,01 par rapport au groupe OVX inf.
La **Figure 5** illustre le poids de la rate (i) des rates témoins (SH), (ii) des rates ovariectomisées et ayant subi une inflammation (OVX inf) ayant reçu un régime alimentaire témoin, (iii) des rates « OVX inf » ayant reçu un régime alimentaire contenant de l'huile d'olive (HO), et (iv) des rates « OVX inf » ayant reçu un régime alimentaire contenant un extrait d'oleuropéine (OL). En ordonnées : poids moyen de le rate +/l'erreur standard par rapport à la moyenne (SEM) +/- l'erreur standard par rapport à la moyenne (SEM). « a » : p< 0,05 par rapport au groupe témoin SH ; « b » : p<0,01 par rapport au groupe OVX inf.

### DESCRIPTION DETAILLEE DE L'INVENTION.

Il est décrit une composition nutritionnelle et une composition pharmaceutique à usage humain ou vétérinaire destinées à stimuler la minéralisation osseuse, en particulier à stimuler la formation de l'os et/ou à inhiber la résorption osseuse, chez l'homme ou l'animal et comprenant, à titre de composé actif, le composé oleuropéine ou l'un de ses dérivés.

L'oleuropéine est un glycoside amer qui est retrouvé notamment dans le fruit, les racines, le tronc et tout particulièrement dans les feuilles des plantes appartenant à la famille des *oleaceae,* et plus spécialement des *Olea europaea.*

L'oleuropéine est connue notamment pour son activité anti-oxydante et anti-inflammatoire, ainsi que pour ses propriétés antifongiques, anti-bactériennes et anti-virales.

L'activité anti-inflammatoire de l'oleuropéine a été mise en évidence du fait de sa propriété de bloquer la flambée respiratoire (« oxidative burst ») des cellules polynucléaires neutrophiles, phénomène à l'origine de la surproduction de radicaux libres lors des réactions inflammatoires de l'organisme.

L'activité anti-virale de l'oleuropéine, ainsi que de certains dérivés de l'oleuropéine, est décrite notamment dans la demande PCT n° WO 96/14064 et dans les brevets américains n° US 6,455,580 et US 6,117,844. L'utilisation de l'oleuropéine pour le traitement d'affections liées au virus de *l'influenza* est décrite dans le brevet américain n° US 6,455,070.

L'utilisation de l'oleuropéine pour la fabrication de compositions pharmaceutiques pour traiter le psoriasis a aussi été décrite, notamment dans le brevet américain n° US 6,440,465.

Owen *et al*. décrivent également que le régime méditerranéen, qui est riche en fruits, en légumes, en fibres, en poissons et en huile d'olive, a un effet protecteur vis-à-vis du cancer, en particulier vis-à-vis du cancer colorectal et du cancer du sein. Il permet de réduire de manière significative la mortalité liée aux maladies cardiaques. Owen *et al.* illustrent plus spécifiquement que les polyphénols présents dans l'huile d'olive - dont l'oleuropéine, le tyrosol et l'hydroxytyrosol - sont des agents antioxydants capables d'inhiber les espèces réactives de l'oxygène (Owen et al., European Journal of Cancer, 2000, 36 1235-1247).

La demande Euro-PCT WO03068171 revendiquant la Priorité de la demande US 60/356847 datée du 13 février 2002 décrit l'utilisation de l'oleuropéine et de l'hydroxytyrosol pour le traitement de pathologies neurologiques liées, entre autres, au SIDA ou pour le traitement de maladies inflammatoires. La demande Euro-PCT WO03068171 cite en tant que maladies inflammatoires, entre autres, l'ostéoarthrite et l'ostéomyélite.

De manière surprenante, il a été montré selon l'invention que l'oleuropéine stimule la formation de l'os et inhibe la résorption osseuse.

Comme cela est illustré par les exemples, l'oleuropéine induit une augmentation de la densité du tissu osseux métaphysaire (os trabéculaire) et de la densité du tissu osseux diaphysaire (os cortical), permettant ainsi une amélioration de la minéralisation osseuse, en particulier dans des modèles expérimentaux d'ostéoporose post-ménopausique et sénile.

Plus spécifiquement, l'oleuropéine inhibe la résorption osseuse chez des rates ovariectomisées, qui est le modèle expérimental animal de référence mimant l'ostéoporose humaine.

On a aussi montré selon l'invention que l'effet d'induction de la minéralisation du tissu osseux provoqué par l'oleuropéine était accompagné d'une activité anti-inflammatoire, comme en témoigne une concentration réduite en orosomucoïde plasmatique, et une absence d'augmentation du poids de la rate, chez les individus ovariectomisés, comme cela est montré dans les exemples.

Cette combinaison de propriétés biologiques surprenante de l'oleuropéine la rend apte notamment à la prévention ou au traitement de l'ostéoporose de type I, mais aussi de l'ostéoporose de type II, qui est souvent accompagnée de réactions inflammatoires.

De plus, comme cela est montré dans les exemples, l'oleuropéine ne présente pas de manière détectable d'effet mimant l'activité d'un composé d'oestrogène, dit aussi effet « estrogen-like », comme en témoignent l'absence de prise de poids des rates traitées, ainsi que l'absence d'effet de l'oleuropéine sur le poids utérin. Ces résultats confirment l'intérêt de l'oleuropéine pour la prévention ou le traitement de tous les troubles du tissu osseux ou pour le maintien de la santé de l'os, chez l'homme ou l'animal, puisque l'activité biologique d'induction de la minéralisation osseuse de l'oleuropéine n'apparaît pas restreinte aux situations dans lesquelles un déficit hormonal est constaté, ou prévu.

Cette activité de minéralisation de l'os induite par l'oleuropéine lui confère une grande utilité en tant que composé actif chez l'homme ou l'animal pour maintenir ou rétablir l'équilibre du métabolisme osseux, c'est-à-dire :
- soit maintenir constante au cours du temps, avec l'âge, les activités des cellules ostéoblastiques et ostéoclastiques et ainsi prévenir des troubles du métabolisme osseux ;
- soit remédier à un déséquilibre du métabolisme osseux, par exemple dans des troubles comme l'ostéoporose, ou encore stimuler la régénération osseuse, par exemple en cas de fêlure ou fracture de l'os.

il est donc décrit l'utilisation du composé oleuropéine ou de l'un de ses dérivés pour la fabrication d'une composition apte à maintenir ou à rétablir l'équilibre du métabolisme osseux chez l'homme ou l'animal, en stimulant la formation de l'os et/ou en inhibant la résorption osseuse.

Il est également décrit l'utilisation du composé oleuropéine ou de l'un de ses dérivés pour la fabrication d'une composition apte à bloquer la déminéralisation du tissu osseux, chez l'homme ou l'animal.

Par « oleuropéine », on entend le composé de formule (I) suivante :

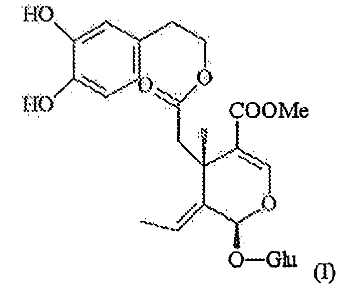

Par « dérivé de l'oleuropéine », on entend respectivement les composés suivants :
(a) l'acide élénolique de formule (II) suivante : 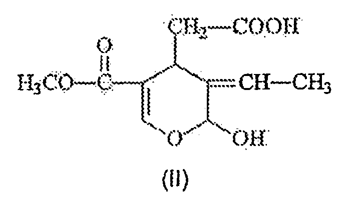
(b) la lactone de formule (III) suivante : 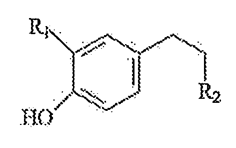 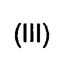 dans laquelle R₂ représente un groupe hydroxyle et R₁ représente l'hydrogène (tyrosol) ou un groupe hydroxyle (hydroxytyrosol);
(c) le composé lactone de formule (IV) suivante : 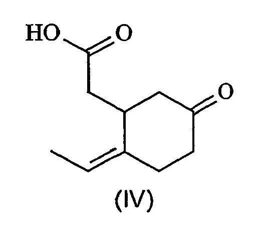
(d) les composés de formule (V) suivante : 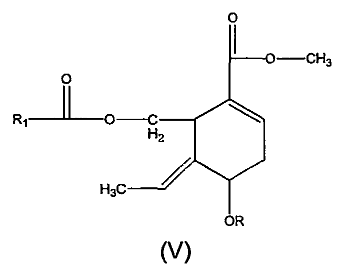 dans laquelle R représente un groupe glycosyl ou un atome d'hydrogène ; R₁ représente le groupe 2-(3,4-dihydroxyphenyl) éthyl, le groupe 2-(4-hydroxyphenyl éthyl), un atome d'hydrogène ou un groupe méthyle ;
(e) l'acide oléanique ;
(f) les produits formés par l'hydrolyse de chacun des composés de formule (I) à (V) ci-dessus dans le tube digestif.

L'acide elenolique et l'hydroxytyrosol sont des dérivés de l'oleuropéine qui résultent de la métabolisation de l'oleuropéine dans l'organisme.

Les composés de formule (V) dans lesquels R₁ représente le groupe 2-(3,4-dihydroxyphenyl) ethyl ou le groupe 2-(4-hydroxyphenyl ethyl) sont retrouvés naturellement dans les plantes de la famille *Olea europea,* par exemple dans les variétés de *Olea europea* dénommées Manzanillo et Mission.

Le dérivé d'oleuropéine de formule (V) dans lequel R₁ représente le groupe 2-(3,4-dihydroxyphenyl) ethyl peut être isolé aisément à partir des feuilles d'olivier (*Olea europaea*) par extraction dans une solution aqueuse ou dans une solution eau/alcool à température de 20°C-25°C, et mieux à une température comprise entre 40°C et 100°C.

L'oleuropéine peut être purifiée, par exemple par des procédés de séparation par chromatographie.

A titre illustratif, l'oleuropéine peut être préparée à partir de feuilles de *Olea europaea* selon l'enseignement du brevet américain n° US 5,714,150, par un procédé comprenant les étapes suivantes :
a) immerger les feuilles de *Olea europaea* avec une solution d'extraction consistant essentiellement en une solution alcoolique contenant au moins 25 % en poids d'alcool, par exemple d'éthanol, pendant une durée d'au moins 4 heures ;
b) récupérer la solution d'extraction obtenue à la fin de l'étape a) ;
c) immerger une seconde fois les feuilles de *Olea europaea* de l'étape a) avec une nouvelle solution d'extraction, de composition identique à celle définie ci-dessus, pendant une durée d'au moins 4 heures ;
d) récupérer la solution d'extraction obtenue à la fin de l'étape c) ;
e) réunir les solutions d'extraction obtenues respectivement à l'étape b) et à l'étape c) ;
f) distiller sous vide la solution obtenue à l'étape e), par exemple à 70°C, afin d'obtenir un concentré contenant de 30% à 40% en poids de solides; et
g) sécher le concentré obtenu à la fin de l'étape f), par exemple par atomisation à 70°C, pour obtenir un extrait sous forme de particules contenant de 30% à 40% en poids du composé oleuropéine.

L'oleuropéine peut aussi être préparée par extraction à partir de feuilles de *Olea europeae* selon l'enseignement de la demande de brevet américain publiée sous le n° US 2003-0017217 le 23 janvier 2003.

L'oleuropéine ou l'un quelconque de ses dérivés peut également être préparé conformément à l'enseignement de la demande PCT n° WO 96/14064 publiée le 17 mai 1996.

Par « stimulation de la formation de l'os », on entend, selon l'invention, la capacité de l'oleuropéine ou de l'un de ses dérivés à stimuler l'activité des ostéoblastes et favoriser ainsi la synthèse de la trame protéique de l'os et le dépôt des minéraux, surtout du calcium, sur cette trame protéique, c'est-à-dire à stimuler la minéralisation de l'os, encore appelée accrétion osseuse.

Pour vérifier que l'apport en oleuropéine à un homme ou un animal, en particulier un mammifère, stimule la formation de l'os, l'homme du métier pourra notamment avoir recours à des mesures conventionnelles de densitométrie et vérifier que l'apport d'oleuropéine ou de l'un de ses dérivés, à une dose donnée, induit un accroissement de la densité osseuse.

Par « inhibition de la résorption osseuse », on entend selon l'invention, une inhibition de l'activité de destruction du tissu osseux par les cellules ostéoclastes. Pour vérifier que l'apport d'oleuropéine ou de l'un de ses dérivés, chez l'homme ou l'animal, inhibe la résorption osseuse, l'homme du métier peut mesurer l'excrétion urinaire de désoxypyridinoline, une diminution de l'excrétion de la désoxypyridinoline étant le reflet d'une inhibition de la résorption osseuse (*in* :Les marqueurs biologiques de remodelage osseux : variations pré-analytique et recommandations pour leur utilisation., P. GARNERO, F. BIANCHI, P.C. CARLIER, V. GENTY, N. JACOB, S.KAMEL, C. KINDERMANS, E. PLOUVIER, M.PRESSAC & J.C. SOUBERBIELLE. Annales de Biologie Clinique, 58 (6), 683-704 (2000)).

L'apport d'oleuropéine ou de l'un de ses dérivés à un organisme animal induit simultanément une stimulation de la formation de l'os et une inhibition de la résorption osseuse, l'accroissement global de la minéralisation osseuse, et donc de la densité de l'os, étant le résultat de l'induction de ces deux mécanismes.

Pour déterminer si un sujet présente un état d'ostéopénie (masse osseuse réduite), et nécessite en conséquence un apport d'oleuropéine ou de l'un de ses dérivés, l'homme du métier pourra se référer notamment au rapport de l'Organisation Mondiale de la Santé (OMS) de 1994 intitulé « Assessment of fracture risk and its application to screening for post-menopausal osteoporosis « WHO Technical Series-843 ».

Une composition nutritionnelle ou une composition thérapeutique contenant de l'oleuropéine ou l'un de ses dérivés comme composé actif est destinée tout d'abord à la prévention de la perte osseuse due à un déséquilibre dans le remodelage du tissu osseux, chez l'homme ou l'animal, en particulier chez un mammifère non humain, notamment un mammifère domestique comme le chien ou le chat, ou encore un cheval.

Une composition telle que définie ci-dessus est également destinée à favoriser la croissance osseuse chez les individus jeunes afin d'optimiser l'acquisition du capital osseux. En particulier, une composition est utile pendant la phase de croissance de l'homme ainsi que des autres mammifères.

La composition contenant de l'oleuropéine ou l'un de ses dérivés est aussi destinée aux individus présentant des symptômes de déficit osseux (ostéopénie), ou susceptibles de souffrir de déficit osseux, c'est-à-dire d'un déséquilibre du rapport entre la formation de l'os et la résorption osseuse lequel, s'il se poursuit, induit une diminution de la masse osseuse. Une composition est également destinée aux individus présentant une expression clinique de la pathologie, à savoir une fracture, ou une maladie dentaire.

En particulier, une composition nutritionnelle ou pharmaceutique à destination humaine ou vétérinaire est utile dans le traitement de pathologies telles que l'ostéoporose de type I ou de type II, les ostéoporoses secondaires, la maladie de Paget, la perte osseuse ou l'ostéolyse observée à proximité d'une prothèse, les maladies de l'os métastatique, l'hypercalcémie due à un cancer, les myélomes multiples, les maladies periodontales ou encore l'ostéoarthrite.

Le fait que l'oleuropéine induise une augmentation non seulement de la densité de l'os trabéculaire, mais également de la densité de l'os cortical démontre le haut niveau d'activité biologique de cette molécule.

En effet, l'os trabéculaire, qui forme la tête de l'os et qui est fortement vascularisé, est le lieu privilégié des échanges de calcium entre l'os et la circulation sanguine, alors que l'os cortical, qui forme le corps droit de l'os, est très peu vascularisé et constitue donc une cible biologique peu accessible aux composés stimulant la formation de l'os et/ou inhibant la résorption osseuse. Pourtant, l'accessibilité de l'os cortical aux composés actifs est importante, du fait que ce tissu osseux, participe pleinement au maintien de la rigidité du squelette (propriétés biomécaniques).

La composition peut se présenter sous la forme d'une composition nutritionnelle ou encore sous la forme d'une composition pharmaceutique, comme cela est décrit ci-après.

### Compositions nutritionnelles contenant de l'oleuropéine ou l'un de ses dérivés.

Comme cela a déjà été mentionné ci-dessus, de nombreux troubles liés à un déséquilibre du métabolisme osseux, telle que l'ostéoporose, évoluent progressivement sur une longue période de temps et nécessitent des traitements chroniques. Leur prévention ou leur traitement peut donc être réalisé grâce à un apport régulier d'oleuropéine ou de l'un de ses dérivés, préférentiellement sous la forme d'une composition nutritionnelle.

De même, un apport nutritionnel régulier d'oleuropéine à des individus jeunes en croissance, hommes ou animaux, est de nature à permettre d'optimiser le pic de masse osseuse et par conséquent la densité minérale osseuse à l'âge adulte.

Egalement, un apport nutritionnel régulier d'oleuropéine est utile pour prévenir la perte osseuse qui se produit au cours du vieillissement.

Il est ainsi décrit une composition nutritionnelle pour stimuler la formation de l'os et/ou inhiber la résorption osseuse, caractérisée en ce qu'elle comprend, à titre de composé nutritionnel actif, le composé oleuropéine ou l'un de ses dérivés.

Par « composition nutritionnelle », on entend, une composition contenant de l'oleuropéine ou l'un de ses dérivés et constituant une composition alimentaire ou encore un supplément alimentaire ne possédant pas les caractéristiques d'un médicament.

Les différentes utilisations du composé oleuropéine ou de l'un de ses dérivés pour la fabrication d'une composition nutritionnelle seront définies ci-après en relation avec les caractéristiques techniques de ladite composition nutritionnelle.

Une composition nutritionnelle est préférentiellement adaptée pour une administration orale.

Selon un premier aspect, une composition nutritionnelle est un aliment diététique utilisé pour le maintien d'une bonne santé de l'homme ou l'animal qui l'ingère. Une telle composition nutritionnelle est également communément désignée « aliment fonctionnel », lequel est destiné à être consommé, soit comme partie intégrante du régime alimentaire, soit comme supplément alimentaire, mais dont le contenu en oleuropéine ou en l'un de ses dérivés implique un rôle physiologique allant au-delà de la fourniture des besoins nutritifs de base.

Selon un premier aspect, ladite composition nutritionnelle est utilisée pour stimuler la formation de l'os chez les individus jeunes en phase de croissance.

Selon un second aspect, ladite composition nutritionnelle est utilisée pour prévenir la perte osseuse qui se produit avec le vieillissement (ostéopénie).

Selon un troisième aspect, que ladite composition nutritionnelle est destinée à prévenir ou traiter des troubles liés à un déséquilibre du rapport entre la formation de l'os et la résorption osseuse.

Selon un quatrième aspect, ladite composition nutritionnelle est destinée à traiter un déficit osseux résultant d'une fracture.

Selon encore un autre aspect, ladite composition nutritionnelle est destinée à prévenir des pathologies choisies parmi l'ostéoporose de type I ou de type II, les ostéopénies secondaires, la maladie de Paget, la perte osseuse ou l'ostéolyse observée à proximité d'une prothèse, les maladies de l'os métastatique, l'hypercalcémie due à un cancer, les myélomes multiples, les maladies periodontales ou l'ostéoarthrite.

Une composition nutritionnelle, caractérisée en ce qu'elle comprend le composé oleuropéine ou l'un de ses dérivés, en tant que composé actif, et se présente sous une grande diversité de formes de compositions alimentaires et de boissons, y compris des jus (de fruits ou de légumes), huiles, beurres, margarines, matières grasses végétales, conserves (par exemple thon à l'huile), soupes, préparations à base de lait (yaourts, fromage blanc), glaces, fromages (par exemple des fromages conservés dans de l'huile), produits cuits (tels que le pain, les biscuits et les gâteaux), desserts, produits de confiserie, barres de céréales, céréales pour le petit déjeuner, condiments, produits d'assaisonnement des aliments (notamment épices et sauces).

Lorsque l'oleuropéine, ou un dérivé de l'oleuropéine, est extrait à partir de la feuille, une composition nutritionnelle est avantageusement utilisée sous la forme d'une solution aqueuse.

Lorsque l'oleuropéine, ou un dérivé de l'oleuropéine, est extrait à partir de l'olive, une composition nutritionnelle est avantageusement utilisée sous la forme d'une solution huileuse.

Une composition nutritionnelle, caractérisée en ce qu'elle comprend le composé oleuropéine ou l'un de ses dérivés, en tant que composé actif, peut aussi se présenter sous la forme d'une grande diversité de produits destinés à l'alimentation animale, qu'ils soient sous forme humide, sous forme semi-humide ou sous forme sèche , notamment sous la forme de croquettes.

Comme cela a déjà été mentionné ci-dessus, l'oleuropéine est un composé majoritaire retrouvé dans les différentes parties, y inclus les feuilles, des plantes de la famille des *Oleaceae*, et plus spécialement dans les feuilles des plantes du genre *Olea europaea* (olivier).

Ainsi, selon un premier aspect préféré, le composé oleuropéine ou l'un de ses dérivés se présente sous la forme d'un produit d'extraction obtenu à partir d'une plante appartenant à la famille des *Oleaceae.* De manière préférée, le produit d'extraction consiste en un produit extrait à partir des tiges, feuilles, des fruits ou des noyaux d'une plante appartenant à la famille des *Oleaceae*.

Préférentiellement, la plante appartenant à la famille des *Oleaceae* est choisie parmi *Olea europaea* (l'olivier), une plante du genre *Ligustrum,* une plante du genre *Syringa,* une plante du genre *Fraximus,* une plante du genre *Jasminum* et une plante du genre *Osmanthus.*

Selon un second aspect préféré, le produit d'extraction consiste en de l'huile d'olive ou en un extrait riche en oleuropéine obtenu à partir de l'huile d'olive.

Selon un troisième aspect préféré, le produit d'extraction consiste en un extrait obtenu à partir des feuilles de *Olea europaea,* qui se présente sous la forme d'un liquide ou sous la forme d'une poudre.

Selon un quatrième aspect, une composition nutritionnelle peut consister en une composition alimentaire enrichie en huile d'olive ou en extrait d'huile d'olive ou de feuilles d'olivier.

Selon un cinquième aspect, une composition nutritionnelle se présente sous la forme de tout produit, en particulier toute boisson, par exemple une boisson aromatisée.

Ainsi, une composition nutritionnelle, qui se présente sous une forme liquide ou sous une forme solide, notamment sous la formé d'une poudre, peut consister en un produit d'extraction obtenu à partir de l'huile d'olive ou des feuilles d'olivier, ou encore comprendre un produit d'extraction obtenu à partir de l'huile d'olive ou des feuilles d'olivier.

Notamment, une composition nutritionnelle se présente sous la forme d'une boisson.

De manière générale, une composition nutritionnelle se présente sous l'une quelconque des formes décrites dans la présente description, et en particulier les aliments à base de matière grasses (beurre, huile, margarine), pain, gâteaux, ou encore de produits alimentaires conservés dans de l'huile, tels que des fromages, des poissons, des viandes, des légumes, des salades) ou encore sous la forme de produit pour l'assaisonnement des aliments, tels que les condiments.

Selon un autre aspect, dans une composition nutritionnelle, l'oleuropéine ou l'un de ses dérivés produit par extraction ou par synthese chimique.

De préférence, une composition nutritionnelle comprend une quantité de composé oleuropéine ou de l'un de ses dérivés adaptée à une administration orale quotidienne comprise entre 0,01 mg et 200 mg.

Pour une consommation chez l'homme, une composition nutritionnelle comprend une quantité de composé actif adaptée à un apport quotidien en oleuropéine ou en l'un de ses dérivés, fourni par ladite composition, comprise entre 0,01 mg et 200 mg, de préférence entre 0,1 mg et 200 mg et de manière tout à fait préférée entre 1 mg et 200 mg.

Pour une consommation par un animal, spécifiquement un mammifère non humain, une composition nutritionnelle est adaptée à une administration quotidienne de composé actif, fournie par ladite composition, comprise entre 1 mg et 200 mg, de préférence 10 mg et 200 mg d'oleuropéine ou l'un de ses dérivés.

Selon encore un autre aspect, la composition nutritionnelle ci-dessus peut comprendre d'autres composés nutritionnels, en combinaison avec l'oleuropéine ou l'un de ses dérivés.

Ainsi, la composition nutritionnelle peut également comprendre une source de calcium, par exemple sous la forme d'un composé organique ou inorganique physiologiquement acceptable, tels que des sels de calcium inorganique (chlorure de calcium, phosphate de calcium, sulfate de calcium, oxyde de calcium, hydroxyde de calcium ou carbonate de calcium) ou des composants organiques contenant du calcium telle que la poudre de lait écrémé, le caséinate de calcium ou encore des sels organiques de calcium (citrate de calcium, maléate de calcium ou leurs mélanges).

La quantité de calcium contenue dans une composition nutritionnelle selon l'invention est adaptée pour une administration quotidienne, fournie par ladite composition, comprise entre 100 mg et 1000 mg, de préférence entre 200 mg et 700 mg et de manière tout à fait préférée entre 300 mg et 600 mg de calcium.

Une composition nutritionnelle selon l'invention peut également comprendre des vitamines, telle que la vitamine A, la vitamine D, la vitamine E, la vitamine K, la vitamine C, l'acide folique, la thiamine, la riboflavine, la vitamine B₆, la vitamine B₁₂, la niacine, la biotine ou encore l'acide pantothénique.

Une composition nutritionnelle peut également comprendre des éléments minéraux et des éléments trace tels que le sodium, le potassium, le phosphore, le magnésium, le cuivre, le zinc, le fer, le sélénium, le chrome et le molybdène.

Elle peut également comprendre des fibres solubles telles que l'agar-agar, un alginate, du caroube, du carragheenan , de la gomme arabique, de la gomme de guar, de la gomme de karaya, de la pectine ou de la gomme xanthane, ces fibres solubles étant sous une forme hydrolysée ou non hydrolysée.

Elle peut aussi comprendre des composés source d'énergie, notamment une ou plusieurs sources d'hydrates de carbone choisis parmi les maltodextrines, l'amidon, le lactose, le glucose, le sucrose, le fructose, le xylitol et le sorbitol et éventuellement les acides gras tels que les oméga-3.

En outre, une composition nutritionnelle peut également comprendre des épices ou encore des herbes aromatiques.

Elle peut également comprendre des composés de type polyphénol, distincts de l'oleuropéine ou de l'un de ses dérivés.

Comme cela a déjà été mentionné précédemment, une composition destinée à stimuler la formation de l'os ou à inhiber la résorption osseuse selon l'invention peut aussi être présentée sous la forme d'une composition pharmaceutique, comme décrit ci-dessous.

### Compositions pharmaceutiques humaines ou vétérinaires selon l'invention.

Il est également décrit une composition pharmaceutique humaine ou vétérinaire pour stimuler la formation de l'os et/ou pour inhiber la résorption osseuse, caractérisée en ce qu'elle comprend, à titre de principe actif, le composé oleuropéine ou l'un de ses dérivés.

En particulier, l'invention concerne l'utilisation de l'oleuropéine ou de l'un de ces dérivés pour la fabrication d'une composition pharmaceutique à usage humain ou vétérinaire pour la prévention ou le traitement d'une pathologie liée à un déséquilibre du métabolisme osseux, c'est-à-dire une composition pharmaceutique apte à stimuler la formation de l'os et/ou à inhiber la résorption osseuse.

Les utilisations de l'oleuropéine pour la fabrication d'une composition pharmaceutique seront décrites en relation avec les caractéristiques techniques de ladite composition pharmaceutique ci-après.

Une composition pharmaceutique comprend, en tant que principe actif, l'oleuropéine ou l'un de ses dérivés, en une quantité adaptée pour stimuler la formation de l'os ou inhiber la résorption osseuse chez des individus nécessitant un tel traitement.

Selon un premier aspect, une composition pharmaceutique est utile pour stimuler la formation de l'os chez les individus jeunes, homme ou animaux, en phase de croissance, afin d'augmenter la densité osseuse atteinte au début de l'âge adulte.

Selon un second aspect, une composition pharmaceutique humaine ou vétérinaire est utile pour prévenir la perte osseuse qui se produit , au cours du vieillissement.

Selon un troisième aspect, une composition pharmaceutique humaine ou vétérinaire est utile pour prévenir ou traiter des troubles ou des pathologies liés à un déséquilibre du rapport entre la formation de l'os et la résorption osseuse.

Selon un quatrième aspect, une composition pharmaceutique humaine ou vétérinaire est utile pour traiter un déficit osseux résultant d'une fracture.

Selon un cinquième aspect, une composition pharmaceutique à destination humaine ou vétérinaire est utile dans le traitement de pathologies liées à un déséquilibre du remodelage osseux, telles que, l'ostéoporose de type I ou de type II, les ostéoporoses secondaires, la maladie de Paget, la perte osseuse ou l'ostéolyse observée à proximité d'une prothèse, les maladies de l'os métastatique, l'hypercalcémie due à un cancer, les myélomes multiples, les maladies periodontales ou encore l'ostéoarthrite.

Il peut s'agir d'une composition pharmaceutique humaine ou vétérinaire, en particulier pour le chien ou le chat, ou encore le cheval.

La composition pharmaceutique se présente sous une forme pour l'administration orale, parentérale, intramusculaire ou intra-veineuse.

Dans sa forme destinée à l'administration humaine, une composition pharmaceutique comprend avantageusement, une quantité d'oleuropéine ou de l'un de ses dérivés adaptée pour une administration quotidienne du composé actif, fournie par ladite composition, comprise entre 0,01 mg et 200 mg.

Sous sa forme destinée à une administration chez l'animal, une composition pharmaceutique comprend une quantité d'oleuropéine ou de l'un de ses dérivés adaptée à une administration quotidienne du composé actif, fourni par ladite composition, comprise entre 1 mg et 200 mg.

Une composition pharmaceutique comprend l'oleuropéine ou l'un de ses dérivés en association avec au moins un excipient choisi dans le groupe constitué par les excipients pharmaceutiquement acceptables.

Des techniques de préparation de compositions pharmaceutiques peuvent être aisément retrouvées par l'homme du métier, par exemple dans l'ouvrage Remmingston's Pharmaceutical Sciences, Mid. Publishing Co, Easton, PA, USA.

Des adjuvants, des véhicules et des excipients physiologiquement acceptables sont aussi décrits dans l'ouvrage intitulé "Handbook of Pharmaceutical Excipients, Seconde édition, American Pharmaceutical Association, 1994.

Pour formuler une composition pharmaceutique, l'homme du métier pourra avantageusement se référer à la dernière édition de la Pharmacopée Européenne ou de la Pharmacopée des Etats-Unis d'Amérique (USP).

L'invention concerne aussi l'utilisation d'un extrait huileux d'oleuropéine comme excipient pour la préparation d'une composition pharmaceutique (combinaison avec d'autres principes actifs, par exemple).

L'homme du métier pourra notamment avantageusement se référer à la quatrième édition « 2002 » de la Pharmacopée Européenne, ou encore à l'édition USP 25-NF 20 de la Pharmacopée Américaine (U.S. Pharmacopeia).

Une composition pharmaceutique telle que définie ci-dessus est adaptée pour une administration orale, parentérale, intramusculaire ou intra-veineuse.

Lorsque la composition pharmaceutique comprend au moins un excipient pharmaceutique ou physiologiquement acceptable, il s'agit en particulier d'un excipient approprié pour une administration de la composition par voie orale ou d'un excipient approprié pour une administration de la composition par voie parentérale.

Il est également décrit une méthode pour prévenir ou traiter un trouble lié à un déséquilibre du métabolisme osseux, en particulier un trouble associé à une perte de la masse osseuse, ladite méthode comprenant une étape au cours de laquelle on administre aux patients une quantité thérapeutiquement efficace d'oleuropéine ou de l'un de ses dérivés ou encore d'une composition pharmaceutique contenant l'oleuropéine ou l'un de ses dérivés.

Une composition pharmaceutique comprenant de l'oleuropéine ou l'un de ses dérivés se présente indifféremment sous une forme solide ou sous une forme liquide.

Pour une administration orale, on préférera une composition pharmaceutique solide, sous la forme de comprimés, de capsules ou de gélules.

Sous la forme liquide, on préférera une composition pharmaceutique sous la forme d'une suspension aqueuse ou d'une suspension lipidique, ou encore sous la forme d'une émulsion eau-dans-huile ou huile-dans-eau.

Des formes pharmaceutiques solides peuvent comprendre, en tant que véhicules, adjuvants ou excipients, au moins un agent diluant, un arôme, un agent solubilisant, un agent lubrifiant, un agent de suspension, un agent liant, un agent désintégrant et un agent d'encapsulation, l'identité et la fonction de ces différents excipients classiques étant documentées complètement dans la Pharmacopée Européenne ou dans la Pharmacopée des Etats-Unis d'Amérique (USP).

De tels composés sont par exemple le carbonate de magnésium, le stéarate de magnésium, le talc, le lactose, la pectine, la dextrine, l'amidon, la gélatine, des matériaux cellulosiques, du beurre de cacao, etc.

Les compositions sous forme liquide peuvent comprendre également de l'eau, le cas échéant en mélange avec du propylène glycol ou du polyéthylène glycol, et éventuellement aussi des agents de coloration, des arômes, des stabilisants et des agents épaississants.

Pour la fabrication d'une composition pharmaceutique, l'oleuropéine, ou l'un de ses dérivés, peut être préparé conformément à l'enseignement des différents documents de brevets cités précédemment dans la description.

L'invention est en outre illustrée , sans pour autant être limitée, par les exemples suivants.

### EXEMPLES

Les résultats des exemples montrent les effets ostéoprotecteurs de l'oleuropéine ou de l'huile d'olive, sur un modèle animal de perte osseuse représentatif de l'ostéoporose sénile par induction d'une inflammation chronique (vieillissement) couplée à une ablation des ovaires (cas de la ménopause).

### A. MATERIEL ET METHODES

L'expérimentation a été conduite sur 40 rates Wistar âgées de 6 mois : trente ovariectomisées (OVX) et 10 pseudo-opérées (SH). Les animaux ont été logés dans des cages métaboliques permettant le recueil séparé des urines à une température contrôlée de 21 ± 1°C, selon un cycle nyctéméral de 12h - 12h. Après l'intervention chirurgicale à JO, les animaux ont été accoutumés pendant sept jours à un régime semi-synthétique de base (INRA Jouy en Josas), additionné de 2,5% d'huile d'arachide et de 2,5% d'huile de colza et humidifié à raison de 1 ml d'eau par gramme de poudre. A l'issue de cette période d'adaptation, les rongeurs ont été répartis sur la base des paramètres pondéraux en 4 lots homogènes et ont reçu quotidiennement pendant 72 jours 22g des régimes suivants :
- Lot SH / 10 rates pseudo-opérées alimentées avec le régime contrôle (régime de base additionné de 2,5% d'huile d'arachide et de 2,5% d'huile de colza),
- Lot OVXinf : 10 rates ovariectomisées ayant reçu le régime contrôle,
- Lot OL : 10 rates ovariéctomisées ayant consommé le régime contrôle additionné de 0,015% d'oleuropéine.
- Lot HO : 10 rates ovariéctomisées auxquelles était distribué le régime de base additionné de 5% d'huile d'olive extra-vierge.
Les huiles ont été stockées à l'abri de la lumière. Les aliments ont été préparés hebdomadairement et stockés à + 4°C.
A J55, soit 3 semaines avant la fin de l'expérimentation, une inflammation a été provoquée dans chaque lot, excepté chez les témoins (SH). Quatre injections sous-cutanées de talc (3,2 g de silicate de magnésium (Sigma) mis en suspension dans 0,71 g/ml de sérum physiologique) ont été administrées en 4. points distincts du dos de l'animal.
Au sacrifice (J80), les animaux ont été anesthésiés par injection intra-péritonéale de chloral (Fluka / solution à 8% ; 0,4 ml/100g de poids vif). Des prélèvements sanguins ont été pratiqués au niveau de l'aorte abdominale pour le dosage de l'orosomucoïde (marqueur de l'inflammation, en particulier de la phase aiguë chez l'homme et le rat (Fournier et al. 2000), produit par le foie et d'autres sites extra-hépatiques sous le contrôle des cytokines II₁, TNFα et II₆).

La rate et l'utérus ont été pesés.

Les fémurs ont été prélevés et débarrassés des tissus mous adjacents, puis conservés dans de l'éthanol à 80% pour la détermination de la densité osseuse.

### B. RESULTATS

### EXEMPLE 1. Poids corporel et utérin (figures 1 et 2).

Le profil pondéral des animaux démontre une évolution similaire, à savoir une augmentation entre le début et la fin de la période expérimentale, quel que soit le groupe considéré. Néanmoins, dès la 2^{ème} semaine, le poids (g) des animaux castrés est supérieur à celui des SH (OVXinf : 294 ± 6 ; SH / 275 ± 6. p = 0,001). Ce phénomène n'est pas corrigé par les deux régimes alimentaires.

Le poids utérin (g utérus/100g poids corporel) est diminué chez les OVxinf (modèle expérimental de l'ostéoporose) (SH / 0,221 ± 0,017 ; OVX/ 0,060 ± 0,003 ; p < 0,001). Il n'est pas restauré par les régimes oleuropéine (0,034 ± 0,001) ou huile d'olive (0,039 ± 0,002) qui sont donc dépourvus d'effet utérotrophique.

### EXEMPLE 2 : Densité minérale osseuse (figure 3).

La castration associée à l'inflammation s'est traduite par une déminéralisation du fémur, mise en évidence par une densité minérale osseuse totale (g/cm²) réduite (SH : 0,2378 ± 0,0050 ; OVXinf : 0,2109 ± 0,0018 p< 0,001). Ce processus est évité (au moins partiellement) par la consommation d'huile d'olive (0,2249 ± 0,0038) ou d'oleuropéine (0,2258 ± 0,0028).

Un profil similaire est démontré au niveau trabéculaire, ainsi que dans l'os cortical (tissu ordinairement peu sensible aux facteurs environnementaux).

### EXEMPLE 3 : Marqueurs de l'inflammation (figues 4 et 5).

La concentration en orosomucoïde (µg/ml) est supérieure chez les OVXinf : 35,9 ± 7,5 ; SH : 17,8 ± 2,4). Ce paramètre est restauré chez les rates ayant reçu le régime huile d'olive (22,8 ± 1,7 p < 0,005). Une tendance similaire (mais non significative) est observée avec le régime oleuropéine.

Le poids de la rate (g rate/100g poids corporel) est augmenté chez les OVXINF (OVX inf : 0,254 ± 0,009 ; SH: 0,219 ± 0,018). Cette splénomégalie est évitée par la prise d'huile d'olive (0,215 ± 0,009, p < 0,01) ou d'oleuropéine (0,201 ± 0,011 p<0,02).

## Revendications (Revendications pour l'(les) Etat(s) contractant(s) suivant(s): AT, BE, CH, DE, DK, ES, FR, GB, GR, IT, LI, LU, MC, NL, SE)

1. Utilisation d'une composition nutritionnelle adaptée pour une administration orale comprenant le composé oleuropéine ou l'un de ses dérivés pour prévenir la perte osseuse qui se produit avec le vieillissement (ostéopénie).

2. Utilisation d'une composition nutritionnelle adaptée pour une administration orale comprenant le composé oleuropéine ou l'un de ses dérivés pour stimuler la formation de l'os chez les individus jeunes en phase de croissance.

3. Utilisation du composé oleuropéine ou de l'un des ses dérivés pour la fabrication d'une composition destinée à traiter ou prévenir les troubles liés à un déséquilibre du rapport entre la formation de l'os et la résorption osseuse chez l'homme ou l'animal, lesdits troubles n'étant pas l'ostéoarthrite et l'ostéomyélite dans le cas où la composition comprend le composé oléuropéine ou l'hydroxytyrosol.

4. Utilisation du composé oleuropéine ou de l'un des ses dérivés pour la fabrication d'une composition destinée à traiter chez l'homme ou l'animal un déficit osseux résultant d'une fracture.

5. Utilisation selon la revendication 3 **caractérisé en ce que** ledit trouble est une pathologie choisie parmi le groupe constitué par l'ostéoporose de type I ou de type II, les ostéoporoses secondaires, la maladie de Paget, la perte osseuse ou l'ostéolyse observée à proximité d'une prothèse, les maladies de l'os métastatique, l'hypercalcémie due à un cancer, les myélomes multiples et les maladies périodontales.

6. Utilisation selon l'une des revendications 1 à 5, **caractérisée en ce que** ladite composition est adaptée à une administration orale d'une quantité quotidienne comprise entre 0,01 à 200 mg du composé oleuropéine ou de l'un de ses dérivés.

7. Utilisation selon l'une quelconque des revendications 1 à 6 **caractérisée en ce que** ladite composition est un supplément alimentaire.

8. Utilisation selon l'une des revendications 1 à 6, caractérisée en ce ladite composition se présente sous forme de compositions alimentaires, de boissons, huiles, beurres, margarines, matières grasses végétales, conserves, soupes, préparations à base de lait, glaces, fromages, produits cuits, desserts, produits de confiserie, barres de céréales, céréales pour le petit déjeuner, condiments, produits d'assaisonnement des aliments.

9. Utilisation selon l'une des revendications 1 à 6, **caractérisée en ce que** ladite composition se présente sous la forme d'un produit destiné à l'alimentation animale, sous forme humide, semi-humide ou sèche.

10. Utilisation selon l'une des revendications 1 à 5 **caractérisée en ce que** la composition est une composition pharmaceutique se présentant sous une forme pour l'administration orale, parentérale, intramusculaire ou intra-veineuse.

11. Utilisation selon l'une des revendications 7 à 9 **caractérisée en ce que** le composé oleuropéine ou l'un de ses dérivés se présente sous la forme d'un produit d'extraction obtenu à partir d'une plante appartenant à la famille des Oleaceae.

12. Utilisation selon la revendication 11 **caractérisée en ce que** le produit d'extraction consiste en un produit extrait à partir des tiges, des feuilles, des fruits ou des noyaux d'une plante appartenant à la famille des Oleaceae.

13. Utilisation selon l'une des revendications 11 et 12 **caractérisée en ce que** la plante appartenant à la famille des Oleaceae est choisie parmi olea europaea, une plante du genre Ligustrum, une plante du genre Syringa, une plante du genre Fraximus, une plante du genre Jasminum et une plante du genre Osmanthus.

14. Utilisation selon l'une des revendications 11 à 13 **caractérisée en ce que** le produit d'extraction consiste en de l'huile d'olive ou en un extrait riche en oleuropéine obtenu à partir de l'huile d'olive, ou des feuilles.

## Revendications (Revendications pour l'(les) Etat(s) contractant(s) suivant(s): PL, RO)

1. Utilisation d'une composition nutritionnelle adaptée pour une administration orale comprenant le composé oleuropéine ou l'un de ses dérivés pour prévenir la perte osseuse qui se produit avec le vieillissement (ostéopénie).

2. Utilisation d'une composition nutritionnelle adaptée pour une administration orale comprenant le composé oleuropéine ou l'un de ses dérivés pour stimuler la formation de l'os chez les individus jeunes en phase de croissance.

3. Utilisation du composé oleuropéine ou de l'un des ses dérivés pour la fabrication d'une composition destinée à traiter ou prévenir les troubles liés à un déséquilibre du rapport entre la formation de l'os et la résorption osseuse chez l'homme ou l'animal.

4. Utilisation du composé oleuropéine ou de l'un des ses dérivés pour la fabrication d'une composition destinée à traiter chez l'homme ou l'animal un déficit osseux résultant d'une fracture.

5. Utilisation selon la revendication 3 **caractérisé en ce que** ledit trouble est une pathologie choisie parmi le groupe constitué par l'ostéoporose de type I ou de type II, les ostéoporoses secondaires, la maladie de Paget, la perte osseuse ou l'ostéolyse observée à proximité d'une prothèse, les maladies de l'os métastatique, l'hypercalcémie due à un cancer, les myélomes multiples et les maladies périodontales et l'ostéoarthrite.

6. Utilisation selon l'une des revendications 1 à 5, **caractérisée en ce que** ladite composition est adaptée à une administration orale d'une quantité quotidienne comprise entre 0,01 à 200 mg du composé oleuropéine ou de l'un de ses dérivés.

7. Utilisation selon l'une quelconque des revendications 1 à 6 **caractérisée en ce que** ladite composition est un supplément alimentaire.

8. Utilisation selon l'une des revendications 1 à 6, caractérisée en ce ladite composition se présente sous forme de compositions alimentaires, de boissons, huiles, beurres, margarines, matières grasses végétales, conserves, soupes, préparations à base de lait, glaces, fromages, produits cuits, desserts, produits de confiserie, barres de céréales, céréales pour le petit déjeuner, condiments, produits d'assaisonnement des aliments.

9. Utilisation selon l'une des revendications 1 à 6, **caractérisée en ce que** ladite composition se présente sous la forme d'un produit destiné à l'alimentation animale, sous forme humide, semi-humide ou sèche.

10. Utilisation selon l'une des revendications 1 à 5 **caractérisée en ce que** la composition est une composition pharmaceutique se présentant sous une forme pour l'administration orale, parentérale, intramusculaire ou intra-veineuse.

11. Utilisation selon l'une des revendications 7 à 9 **caractérisée en ce que** le composé oleuropéine ou l'un de ses dérivés se présente sous la forme d'un produit d'extraction obtenu à partir d'une plante appartenant à la famille des Oleaceae.

12. Utilisation selon la revendication 11 **caractérisée en ce que** le produit d'extraction consiste en un produit extrait à partir des tiges, des feuilles, des fruits ou des noyaux d'une plante appartenant à la famille des Oleaceae.

13. Utilisation selon l'une des revendications 11 et 12 **caractérisée en ce que** la plante appartenant à la famille des Oleaceae est choisie parmi olea europaea, une plante du genre Ligustrum, une plante du genre Syringa, une plante du genre Fraximus, une plante du genre Jasminum et une plante du genre Osmanthus.

14. Utilisation selon l'une des revendications 11 à 13 **caractérisée en ce que** le produit d'extraction consiste en de l'huile d'olive ou en un extrait riche en oleuropéine obtenu à partir de l'huile d'olive, ou des feuilles.

## Claims (Claims for the following Contracting State(s): AT, BE, CH, DE, DK, ES, FR, GB, GR, IT, LI, LU, MC, NL, SE)

1. Use of a nutritional composition suitable for oral administration comprising oleuropein compound or a derivative thereof for preventing bone loss that occurs with aging (osteopenia).

2. Use of a nutritional composition suitable for oral administration comprising oleuropein compound or a derivative thereof for stimulating bone formation in young individuals being in growth period.

3. Use of the compound oleuropein or a derivative thereof for preparing a composition to be used for treating or preventing the disorders associated with an unbalanced ratio between bone formation and bone resorption in a human or an animal, with the proviso that the said disorders are not osteoarthritis and osteomyelitis when the said composition comprises the compound oleuropein or hydroxytyrosol.

4. Use of the compound oleuropein or a derivative thereof for preparing a composition to be used for treating a bone deficiency resulting from a fracture in a human or an animal.

5. The use according to claim 3 wherein the said disorder is selected from the group consisting of type I or type II osteoporosis, secondary osteoporosis, Paget's disease, bone loss or osteolysis observed at the vicinity of a prosthesis, metastatic bone diseases, cancer induced hypercalcemia, multiple myeloma and periodontal diseases.

6. The use according to anyone of claims 1 to 5 wherein the said composition is suitable for the oral administration of a daily amount of oleuropein compound or a derivative thereof ranging from 0.01 to 200 mg.

7. The use according to anyone of claims 1 to 6 wherein the said composition is a dietary complement.

8. The use according to anyone of claims 1 to 6 wherein the said composition comes as food compositions, beverages, oils, butters, margarines, vegetal fats, cans, soups, milk-based preparations, ice creams, cheeses, baked products, desserts, confectionery products, cereal bars, breakfast cereals, condiments, flavouring products.

9. The use according to anyone of claims 1 to 6 wherein the said composition is a product for animal feeding, in a wet, half-wet or dry form.

10. The use according to anyone of claims 1 to 5 wherein the said composition is a pharmaceutical composition for administration by oral, parenteral, intramuscular or intravenous route.

11. The use according to anyone of claims 7 to 9 wherein the oleuropein compound or one derivative thereof comes as an extraction product from a plant belonging to the Oleaceae family.

12. The use according to claim 11 wherein the extraction product is a product extracted from the stems, the leaves, the fruits or the stones of a plant belonging to the Oleaceae family.

13. The use according to anyone of claims 11 and 12 wherein the plant belonging to the Oleaceae family is selected from the group consisting of olea europaea, a plant of genus Ligustrum, a plant of genus Syringa, a plant of genus Fraximus, a plant of genus Jasminu and a plant of genus Osmanthus.

14. The use according to anyone of claims 11 to 13 wherein the extraction product is an olive oil or an oleuropein-enriched extract from olive oil or leaves.

## Claims (Claims for the following Contracting State(s): PL, RO)

1. Use of a nutritional composition suitable for oral administration comprising oleuropein compound or a derivative thereof for preventing bone loss that occurs with aging (osteopenia).

2. Use of a nutritional composition suitable for oral administration comprising oleuropein compound or a derivative thereof for stimulating bone formation in young individuals being in growth period.

3. Use of the compound oleuropein or a derivative thereof for preparing a composition to be used for treating or preventing the disorders associated with an unbalanced ratio between bone formation and bone resorption in a human or an animal, with the proviso that the said disorders are not osteoarthritis and osteomyelitis.

4. Use of the compound oleuropein or a derivative thereof for preparing a composition to be used for treating a bone deficiency resulting from a fracture in a human or an animal.

5. The use according to claim 3 wherein the said disorder is selected from the group consisting of type I or type II osteoporosis, secondary osteoporosis, Paget's disease, bone loss or osteolysis observed at the vicinity of a prosthesis, metastatic bone diseases, cancer induced hypercalcemia, multiple myeloma, periodontal diseases and osteoarthritis.

6. The use according to anyone of claims 1 to 5 wherein the said composition is suitable for the oral administration of a daily amount of oleuropein compound or a derivative thereof ranging from 0.01 to 200 mg.

7. The use according to anyone of claims 1 to 6 wherein the said composition is a dietary complement.

8. The use according to anyone of claims 1 to 6 wherein the said composition comes as food compositions, beverages, oils, butters, margarines, vegetal fats, cans, soups, milk-based preparations, ice creams, cheeses, baked products, desserts, confectionery products, cereal bars, breakfast cereals, condiments, flavouring products.

9. The use according to anyone of claims 1 to 6 wherein the said composition is a product for animal feeding, in a wet, half-wet or dry form.

10. The use according to anyone of claims 1 to 5 wherein the said composition is a pharmaceutical composition for administration by oral, parenteral, intramuscular or intravenous route.

11. The use according to anyone of claims 7 to 9 wherein the oleuropein compound or one derivative thereof comes as an extraction product from a plant belonging to the Oleaceae family.

12. The use according to claim 11 herein the extraction product is a product extracted from the stems, the leaves, the fruits or the stones of a plant belonging to the Oleaceae family.

13. The use according to anyone of claims 11 and 12 wherein the plant belonging to the Oleaceae family is selected from the group consisting of olea europaea, a plant of genus Ligustrum, a plant of genus Syringa, a plant of genus Fraximus, a plant of genus Jasminum and a plant of genus Osmanthus.

14. The use according to anyone of claims 11 to 13 wherein the extraction product is an olive oil or an oleuropein-enriched extract from olive oil or leaves.

## Patentansprüche (Patentansprüche für folgende(n) Vertragsstaat(en): AT, BE, CH, DE, DK, ES, FR, GB, GR, IT, LI, LU, MC, NL, SE)

1. Verwendung einer für eine orale Verabreichung angepassten Nahrungszusammensetzung, die die Verbindung Oleuropein oder eines ihrer Derivate umfasst, für die Prävention des Knochenverlusts, der mit der Alterung einhergeht (Osteopenie).

2. Verwendung einer für eine orale Verabreichung angepassten Nahrungszusammensetzung, die die Verbindung Oleuropein oder eines ihrer Derivate umfasst, zum Stimulieren der Knochenbildung bei jungen Individuen in der Wachstumsphase.

3. Verwendung der Verbindung Oleuropein oder eines ihrer Derivate zur Herstellung einer Zusammensetzung zur Behandlung oder Prävention der Störungen, die mit einem Ungleichgewicht zwischen Knochenbildung und Knochenresorption bei Mensch oder Tier verbunden sind, mit der Maßgabe, ass es sich bei den Störungen nicht um Osteoarthritis oder Osteomyelitis handelt, wenn die Zusammensetzung die Verbindung Oleuropein oder Hydroxytyrosol umfasst.

4. Verwendung der Verbindung Oleuropein oder eines ihrer Derivate zur Herstellung einer Zusammensetzung zur Behandlung eines sich aus einer Fraktur ergebenden Knochenmangels bei Mensch oder Tier.

5. Verwendung gemäß Anspruch 3, **dadurch gekennzeichnet, dass** die Störung eine Erkrankung ist, die aus der Gruppe ausgewählt ist, die aus Osteoporose Typ I oder Typ II, sekundären Osteoporosen, Morbus Paget, Knochenverlust oder Osteolyse, der bzw. die in der Nähe einer Prothese beobachtet wird, metastatische Knochenkrankheiten, Hypercalcämie aufgrund einer Krebserkrankung, multiplen Myelomen und Erkrankungen des Zahnhalteapparats besteht.

6. Verwendung gemäß einem der Ansprüche 1 bis 5, **dadurch gekennzeichnet, dass** die Zusammensetzung für eine orale Verabreichung in einer Tagesdosis zwischen 0,01 und 200 mg der Verbindung Oleuropein oder eines ihrer Derivate angepasst ist.

7. Verwendung gemäß einem der Ansprüche 1 bis 6, **dadurch gekennzeichnet**, ass die Zusammensetzung ein Nahrungsergänzungsmittel ist.

8. Verwendung gemäß einem der Ansprüche 1 bis 6, **dadurch gekennzeichnet, dass** die Zusammensetzung in Form von Nahrungszusammensetzungen, Getränken, Ölen, Butter, Margarine, pflanzlichen Fetten, Konserven, Suppen, Präparaten auf Milchbasis, Eis, Käse, gekochten Produkten, Desserts, Konditoreiprodukten, Getreideriegeln, Getreide für das Frühstück, Gewürzen, Produkten zum Würzen von Nahrungsmitteln vorliegt.

9. Verwendung gemäß einem der Ansprüche 1 bis 6, **dadurch gekennzeichnet, dass** die Zusammensetzung in Form eines Produkts, das für die Ernährung von Tieren bestimmt ist, in feuchter, halbfeuchter oder trockener Form vorliegt.

10. Verwendung gemäß einem der Ansprüche 1 bis 5, **dadurch gekennzeichnet, dass** die Zusammensetzung eine pharmazeutische Zusammensetzung ist, die in einer Form für die orale, parenterale, intramuskuläre oder intravenöse Verabreichung vorliegt.

11. Verwendung gemäß einem der Ansprüche 7 bis 9, **dadurch gekennzeichnet, dass** die Verbindung Oleuropein oder eines ihrer Derivate in Form eines Extraktionsprodukts vorliegt, das aus einer zur Familie der Oleaceae gehörenden Pflanze erhalten wird.

12. Verwendung gemäß Anspruch 11, **dadurch gekennzeichnet, dass** das Extraktionsprodukt aus einem Produkt besteht, das aus Stängeln, Blättern, Früchten oder Nüssen einer zur Familie der Oleaceae gehörenden Pflanze extrahiert wurde.

13. Verwendung gemäß einem der Ansprüche 11 und 12, **dadurch gekennzeichnet, dass** die zur Familie der Oleaceae gehörende Pflanze aus *Olea europaea,* einer Pflanze der Gattung *Ligustrum,* einer Pflanze der Gattung *Syringa,* einer Pflanze der Gattung *Fraxinus,* einer Pflanze der Gattung *Jasminum* und einer Pflanze der Gattung *Osmanthus* ausgewählt ist.

14. Verwendung gemäß einem der Ansprüche 11 bis 13, **dadurch gekennzeichnet, dass** das Extraktionsprodukt aus Olivenöl oder einem Oleuropein-reichen Extrakt, der aus Olivenöl oder Blättern erhalten wurde, besteht.

## Patentansprüche (Patentansprüche für folgende(n) Vertragsstaat(en): PL, RO)

1. Verwendung einer für eine orale Verabreichung angepassten Nahrungszusammensetzung, die die Verbindung Oleuropein oder eines ihrer Derivate umfasst, für die Prävention des Knochenverlusts, der mit der Alterung einhergeht (Osteopenie).

2. Verwendung einer für eine orale Verabreichung angepassten Nahrungszusammensetzung, die die Verbindung Oleuropein oder eines ihrer Derivate umfasst, zum Stimulieren der Knochenbildung bei jungen Individuen in der Wachstumsphase.

3. Verwendung der Verbindung Oleuropein oder eines ihrer Derivate zur Herstellung einer Zusammensetzung zur Behandlung oder Prävention der Störungen, die mit einem Ungleichgewicht zwischen Knochenbildung und Knochenresorption bei Mensch oder Tier verbunden sind.

4. Verwendung der Verbindung Oleuropein oder eines ihrer Derivate zur Herstellung einer Zusammensetzung zur Behandlung eines sich aus einer Fraktur ergebenden Knochenmangels bei Mensch oder Tier.

5. Verwendung gemäß Anspruch 3, **dadurch gekennzeichnet, dass** die Störung eine Erkrankung ist, die aus der Gruppe ausgewählt ist, die aus Osteoporose Typ I oder Typ II, sekundären Osteoporosen, Morbus Paget, Knochenverlust oder Osteolyse, der bzw. die in der Nähe einer Prothese beobachtet wird, metastatische Knochenkrankheiten, Hypercalcämie aufgrund einer Krebserkrankung, multiplen Myelomen und Erkrankungen des Zahnhalteapparats sowie Osteoarthritis besteht.

6. Verwendung gemäß einem der Ansprüche 1 bis 5, **dadurch gekennzeichnet, dass** die Zusammensetzung für eine orale Verabreichung in einer Tagesdosis zwischen 0,01 und 200 mg der Verbindung Oleuropein oder eines ihrer Derivate angepasst ist.

7. Verwendung gemäß einem der Ansprüche 1 bis 6, **dadurch gekennzeichnet, dass** die Zusammensetzung ein Nahrungsergänzungsmittel ist.

8. Verwendung gemäß einem der Ansprüche 1 bis 6, **dadurch gekennzeichnet, dass** die Zusammensetzung in Form von Nahrungszusammensetzungen, Getränken, Ölen, Butter, Margarine, pflanzlichen Fetten, Konserven, Suppen, Präparaten auf Milchbasis, Eis, Käse, gekochten Produkten, Desserts, Konditoreiprodukten, Getreideriegeln, Getreide für das Frühstück, Gewürzen, Produkten zum Würzen von Nahrungsmitteln vorliegt.

9. Verwendung gemäß einem der Ansprüche 1 bis 6, **dadurch gekennzeichnet**, dans die Zusammensetzung in Form eines Produkts, das für die Ernährung von Tieren bestimmt ist, in feuchter, halbfeuchter oder trockener Form vorliegt.

10. Verwendung gemäß einem der Ansprüche 1 bis 5, **dadurch gekennzeichnet, dass** die Zusammensetzung eine pharmazeutische Zusammensetzung ist, die in einer Form für die orale, parenterale, intramuskuläre oder intravenöse Verabreichung vorliegt.

11. Verwendung gemäß einem der Ansprüche 7 bis 9, **dadurch gekennzeichnet**, dans die Verbindung Oleuropein oder eines ihrer Derivate in Form eines Extraktionsprodukts vorliegt, as aus einer zur Familie der Oleaceae gehörenden Pflanze erhalten wird.

12. Verwendung gemäß Anspruch 11, **dadurch gekennzeichnet, dass** das Extraktionsprodukt aus einem Produkt besteht, das aus Stängeln, Blättern, Früchten oder Nüssen einer zur Familie der Oleaceae gehörenden Pflanze extrahiert wurde.

13. Verwendung gemäß einem der Ansprüche 11 und 12, **dadurch gekennzeichnet, dass** die zur Familie der Oleaceae gehörende Pflanze aus *Olea europaea*, einer Pflanze der Gattung *Ligustrum,* einer Pflanze der Gattung *Syringa,* einer Pflanze der Gattung *Fraxinus,* einer Pflanze der Gattung *Jasminum* und einer Pflanze der Gattung *Osmanthus* ausgewählt ist.

14. Verwendung gemäß einem der Ansprüche 11 bis 13, **dadurch gekennzeichnet, dass** das Extraktionsprodukt aus Olivenöl oder einem Oleuropein-reichen Extrakt, der aus Olivenöl oder Blättern erhalten wurde, besteht.
